# EUROPÄISCHE PATENTSCHRIFT

(11) **EP 2 996 737 B1**
(45) Veröffentlichungstag und Bekanntmachung des Hinweises auf die Patenterteilung: **18.12.2019**
(21) Anmeldenummer: 14724058.4
(22) Anmeldetag: 08.05.2014
(51) Int. Cl.: A61M 1/14, G16H 40/63, G16H 50/20

(54) **VORRICHTUNG UND VERFAHREN ZUR BEREITSTELLUNG VON BEHANDLUNGSPARAMETERN FÜR DIE BEHANDLUNG EINES PATIENTEN**
DEVICE AND METHOD FOR MAKING AVAILABLE TREATMENT PARAMETERS FOR THE TREATMENT OF A PATIENT
DISPOSITIF ET PROCÉDÉ DE CHARGEMENT DE PARAMÈTRES DE TRAITEMENT D'UN PATIENT

(30) Priorität: 17.05.2013 DE 102013008418
(43) Veröffentlichungstag der Anmeldung: 23.03.2016
(73) Patentinhaber: Fresenius Medical Care Deutschland GmbH, 61352 Bad Homburg (DE)
(72) Erfinder: EIFLER, Peter, 61118 Bad Vilbel (DE); GRÄFE, Marco, 61350 Bad Homburg (DE); STEIL, Helmut, 63571 Gelnhausen (DE)
(74) Vertreter: Dreyhsig, Jörg
(86) Internationale Anmeldenummer: PCT/EP2014/059448
(87) Internationale Veröffentlichungsnummer: WO 2014/184087

(56) Entgegenhaltungen:
- EP-A1- 1 195 708
- DE-A1-102004 011 264
- DE-A1-102005 025 516
- US-A1- 2005 086 072
- US-A1- 2010 010 425
- US-B2- 7 072 769

## Beschreibung

### Technisches Gebiet

Die Erfindung betrifft die Bereitstellung von Behandlungsparametern für die Behandlung eines Patienten, insbesondere von Behandlungsparametern für eine Dialysebehandlung eines Dialysepatienten.

### Hintergrund

Im klinischen Alltag werden verstärkt Klinikdatenmanagementsysteme eingesetzt. Diese dienen der Verwaltung, Bereitstellung und Verarbeitung von Patientendaten, Behandlungsdaten, Diagnosedaten und anderen Daten, die bei der Behandlung von Patienten anfallen oder benötigt werden. Ein Beispiel für den Einsatz von Klinikdatenmanagementsystemen sind Dialysekliniken zur Versorgung chronisch niereninsuffizienter Patienten.

Die Hämodialyse ist ein extrakorporales Verfahren zur Blutreinigung bei chronisch niereninsuffizienten Patienten.

Bei der Hämodialyse wird Blut in einem extrakorporalen Kreislauf kontinuierlich einem Patienten entnommen, durch einen Hämodialysator geleitet und dem Patienten wieder reinfundiert. Dabei wird ein Stoffaustausch durchgeführt, der dem der Nieren ähnlich ist. Der Hämodialysator besteht aus zwei durch eine semipermeable Membran getrennten Kammern, von denen die eine vom Blut und die andere von einer Reinigungsflüssigkeit - der Dialysierflüssigkeit - durchflossen wird. Die handelsüblichen Hämodialysatoren weisen hierfür meist Tausende von Hohlfasern auf, deren Wände semipermeabel für die auszutauschenden Substanzen sind. Das Blut wird durch den Innenraum der Hohlfasern geleitet, während die Dialysierflüssigkeit in meist gegenläufiger Richtung in den Faserzwischenraum eingespeist und abgeführt wird.

Die Dialysierflüssigkeit weist Konzentrationen von Blutinhaltsstoffen wie Elektrolyten auf, die in etwa denen eines Gesunden entsprechen, damit die entsprechenden Konzentrationen im Blut auf einem normalen Niveau gehalten werden können. Für die Konzentration eines oder mehrer Elektrolyten in der Dialysierflüssigkeit über den Verlauf der Behandlung kann ein zeitabhängiges Konzentrationsprofil vorgegeben werden, das für eine bestimmte Behandlung anzuwenden ist. So wird typischerweise ein Konzentrationsprofil für die Natriumionenkonzentration vorgegeben, das als Natriumprofil bezeichnet wird.

Aus dem Blut zu entfernende Stoffe wie zum Beispiel Kreatinin oder beta2-Mikroglobulin sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen. Für die Ultrafiltrationsrate über den Verlauf der Behandlung kann ein zeitabhängiges Ultrafiltrationsprofil vorgegeben werden, das für eine bestimmte Behandlung anzuwenden ist.

Heutige Datenmanagementsysteme im Bereich der Dialyse sind modular aufgebaut und nutzen aufeinander abgestimmte Systemkomponenten, um die individuellen Anforderungen und Arbeitsaufgaben der Anwender abdecken zu können. Sie bestehen aus einem an die Dialysemaschinen angebundenen System (Monitoringsystem) sowie einem System zur Verwaltung von patientenbezogenen Datensätzen (Clinical Management System). Die Behandlungsverschreibungen für die Therapie der Patienten werden hierbei im Clinical Management System durchgeführt und danach über das Monitoringsystem zur automatisierten Voreinstellung der Dialysegeräte mit Patienten- und Behandlungsspezifischen Daten genutzt. In diesem Prozess müssen die Datenformate einer bestimmten für die Dialysebehandlung vorgesehenen Dialysemaschine in dem Clinical Management System bekannt sein. So müssen die für eine Dialysebehandlung vorgesehen Behandlungsparameter auf den für die Behandlung vorgesehenen Maschinentyp abgestimmt sein. Ändert sich etwa aus organisatorischen Gründen der für eine Behandlung eines bestimmten Patienten vorgesehene Maschinentyp, so muss eine Neukonfiguration der Behandlungsparameter vorgenommen werden.

Aus DE 10 2005 025 516 ist ein Behandlungssystem bekannt, das eine Dialysevorrichtung und einen Server zur Bereitstellung von patientenbezogenen Daten umfasst. Die vom Server geladenen patientenbezogenen Daten werden auf einer Anzeigevorrichtung angezeigt, auf der auch maschinenbezogene Datensätze angezeigt werden. Es ist daher eine Aufgabe der vorliegenden Erfindung, ein Verfahren sowie ein System bereitzustellen, das eine flexible Konfiguration von Behandlungsparametern ermöglicht.

### Zusammenfassung

Diese Aufgabe wird gelöst durch ein Verfahren zum Bereitstellen von Behandlungsparametern zum Kontrollieren oder Steuern einer Dialysemaschine nach Anspruch 1 sowie durch ein System nach Anspruch 11. Vorteilhafte Ausführungsformen sind in den abhängigen Ansprüchen angegeben.

### Kurzbeschreibung der Zeichnungen

Figur 1 zeigt ein Blockschaltbild eines Datenmanagementsystems im Einklang mit der offenbarungsgemäßen Lehre.
Figur 2 zeigt ein Flussdiagramm eines Verfahrens in einem Datenmanagementsystem im Einklang mit der offenbarungsgemäßen Lehre.
Figur 3 zeigt ein Flussdiagramm eines weiteren Verfahrens in einem Datenmanagementsystem im Einklang mit der offenbarungsgemäßen Lehre.

### Detaillierte Beschreibung der Zeichnungen

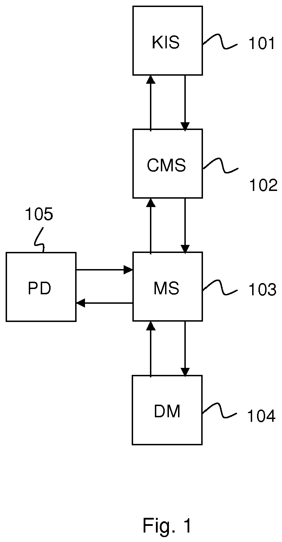

Figur 1 zeigt ein Datenmanagementsystem für den Einsatz im Bereich der Dialyse. Ein Datenmanagementsystem im Bereich der Dialyse ist typischerweise mit einem Krankenhausinformationssystem 101 verbunden, zur Handhabung allgemeiner Patientendaten der Dialysepatienten, etwa Abrechnungsdaten, oder Behandlungs- oder diagnostischer Daten des Patienten, die keinen unmittelbaren Bezug zu seiner Dialysebehandlung haben. Das Krankenhausinformationssystem 101 ist zum bidirektionalen Datenaustausch mit einem Clinical Managementsystem 102, zur Handhabung von Patientendatensätzen, die unmittelbaren Bezug zur Dialysebehandlung haben, etwa Trenddaten, wie Trends der Daten zum Fluidmanagement, etwa Trockengewicht, Überwässerung, Eisenhaushalt usw. Diese können beispielsweise der Langzeitdokumentation dienen. Das Clinical Managementsystem 101 dient dem Arzt oder sonstigem Behandler als Schnittstelle für die Eingabe von Behandlungsverschreibungen, etwa in Bezug auf das Fluidmanagement eines Patienten, wie Ultrafiltrationsprofile oder Gewichtsabnahmeprofile oder die über den Behandlungsverlauf zu verwendenden Konzentrationsprofile der Dialysierflüssigkeit, wie etwa das Natriumprofil, das den zeitlichen Verlauf der Natriumkonzentration über die Behandlung oder zu anderen Elektrolytprofilen. Der Begriff des Behandlungsprofils soll im Folgenden auf das Fluidmanagement bezogene Profile wie Ultrafiltrationsprofile oder Gewichtsabnahmeprofile als auch Konzentrationsprofile wie etwa Elektrolytprofile umfassen. Das Clinical Management System 102 ist zur bidirektionalen Datenkommunikation mit einem Monitoringsystem 103 verbunden, von dem die Dialysemaschinen 104 überwacht und gesteuert werden. Das Monitoringsystem 103 ist wiederum mit periphären Geräten 105 wie Waagen oder Bioimpedanzmessgeräten verbunden, die über diese Verbindung Untersuchungsdaten an das Monitoringsystem 103 senden. Von dem Monitoringsystem 103 werden die Dialysemaschinen 104 angesteuert, wobei die von den periphären Geräten ermittelten Behandlungsdaten verwendet werden können.

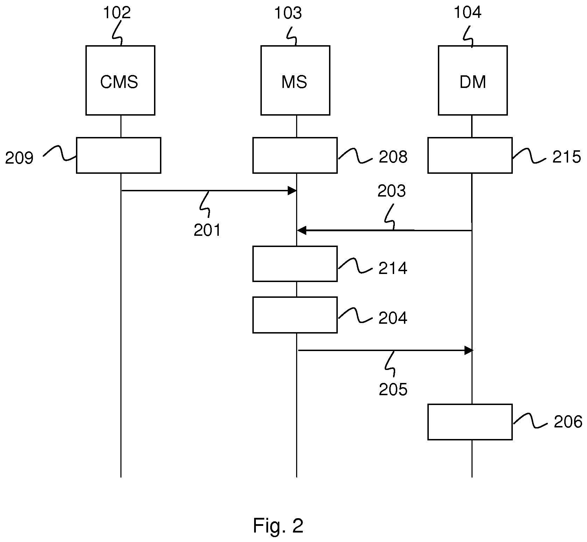

Fig 2 ist ein Flussdiagramm für einen Nachrichtenfluss sowie eine Abfolge von Verarbeitungsschritten im Zusammenhang mit dem in Figur 1 dargestellten Datenmanagementsystem. Die Elemente des Datenmanagementsystems sind mit gleichen Bezugsziffern wiedergegeben.

Der Behandlung geht ein Eingabeschritt 208 voraus zur Eingabe von maschinenbezogenen Behandlungsparametern, die unabhängig von der Behandlung eines bestimmten Patienten sind.

Der Behandlung geht außerdem ein Eingabeschritt 209 voraus, zur Eingabe eines patientenbezogenen Datensatzes von patientenbezogenen Behandlungsparametern eines zu behandelnden Patienten, oder mit anderen Worten: der Verschreibung für den zu behandelnden Patienten. Für die Verschreibung wird ein patientenbezogener Datensatz oder mit anderen Worten ein Verschreibungsdatensatz angelegt. Der Verschreibungsdatensatz enthält Vorgaben für die durchzuführende Dialysebehandlung in einer von der Dialysemaschine oder deren Maschinentyp unabhängigen Form. So können für die Ultrafiltration Behandlungsparameter für die gesamte Behandlung vorgegeben werden wie etwa die Ultrafiltrationsrate zu Beginn der Behandlung, die Ultrafiltrationsrate zum Ende der Behandlung, die insgesamt zu entziehende Ultrafiltrationsmenge die maximale Ultrafiltrationsrate, die minimale Ultrafiltrationsrate oder die durchschnittliche Ultrafiltrationsrate, In ähnlicher Weise kann für eine Elektrolytkonzentration, etwa eine Natriumkonzentration ein Behandlungsparameter für die gesamte Behandlung vorgegeben werden, etwa eine Natriumkonzentration zu Beginn der Behandlung, eine Natriumkonzentration zum Ende der Behandlung, eine maximale, eine minimale oder eine durchschnittliche Natriumkonzentration oder ein über die Behandlungsdauer integrierter oder aufsummierter Wert der Natriumkonzentration oder eine Gesamtmenge.

In einer Ausführungsform kann darüber hinaus der Grundtyp eines Behandlungsprofils vorgegeben werden etwa konstantes Profil, ansteigendes Profil, abnehmendes Profil, Stufenprofil, Rampenprofil, Kammprofil, bei dem zwischen zwei verschiedenen Werten umgeschaltet wird. Auf diese Weise kann eine bestimmte Eigenschaft eines Behandlungsprofils vorgegeben werden, ohne dass bereits eine Auswahl des für die Dialysebehandlung anzuwendenden Behandlungsprofils vorgenommen wurde. So kann etwa vorgegeben werden, dass ein ansteigendes Profil verwendet werden soll, wobei offengelassen wird, ob es als mit konstanter Steigung ansteigendes Profil oder ein Stufenprofil realisiert werden soll oder in welcher zeitlichen Abfolge Stufen vorzusehen sind.

Für einen bestimmten Typ von Dialysemaschinen ist typischerweise eine bestimmte Anzahl von Grundtypen von Behandlungsprofilen oder Grundprofilen möglich.

Darüber hinaus können mehrere Grundtypen von Behandlungsprofilen vorgegeben werden, wobei eine Abfolge unterschiedlicher Priorität für die verschiedenen Grundtypen angegeben wird, etwa mit erster Priorität ein mit konstanter Rate ansteigendes Profil, mit zweiter Priorität ein ansteigendes Stufenprofil usw.

Die patientenbezogenen Behandlungsdaten werden von dem Clinical Management System 102 in der Nachricht 201 an das Monitoringsystem 103 übertragen.

In einem Auswahlschritt erfolgt die Auswahl der für die Dialysebehandlung vorgesehenen Dialysemaschine 104, etwa in dem eine Pflegekraft den Patienten an der vorgesehenen Dialysemaschine anmeldet. In der Nachricht 203 übermittelt die Dialysemaschine 104 an das Monitoringsystem 103 eine Identifikation, die es erlaubt, den Typ der für die Dialysebehandlung des Patienten vorgesehenen Dialysemaschine 104 zu bestimmen.

Anhand der Identifikation der Dialysemaschine erfolgt die Auswahl eines maschinenbezogenen Datensatzes für eine bestimmte Dialysebehandlung des Patienten in einem Verarbeitungsschritt 214.

In einem Verarbeitungsschritt 204 erfolgt die Zusammenstellung eines Behandlungsdatensatzes zum Festlegen von Behandlungsparametern für die durchzuführende Dialysebehandlung, unter Verwendung des maschinenbezogenen Datensatzes sowie des patientenbezogenen Datensatzes.

So kann anhand des für die Behandlung vorgesehenen Maschinentyps, des Grundtyps des Behandlungsprofils und des Behandlungsparameters das während der Behandlung zu verwendende Behandlungsprofil bestimmt werden.

Dazu kann etwa anhand des Grundtyps des Behandlungsprofils und des Maschinentyps zunächst ein Grundprofil etwa ein dimensionsloses oder normiertes Profil gebildet werden, zum Beispiel anhand eines ansteigenden Profils, ein Stufenprofil mit einer bestimmten Anzahl von Stufen, entsprechend dem Maschinentyp.

Sodann kann das für die Behandlung anzuwendende Behandlungsprofil aus dem Grundprofil und dem Behandlungsparameter gebildet werden, z.B. durch Multiplikation eines Durchschnittswertes mit einem dimensionslosen oder genormten Grundprofil.

Das für die Behandlung anzuwendende Behandlungsprofil wird in einen Behandlungsdatensatz eingebunden, der Behandlungsparameter für die durchzuführende Dialysebehandlung festlegt. Der Behandlungsdatensatz wird in einer Nachricht 205 an die Dialysemaschine versandt, die in einem Behandlungsschritt 206 eine Dialysebehandlung unter Verwendung der Behandlungsparameter, insbesondere der anzuwendenden Behandlungsprofile durchführt.

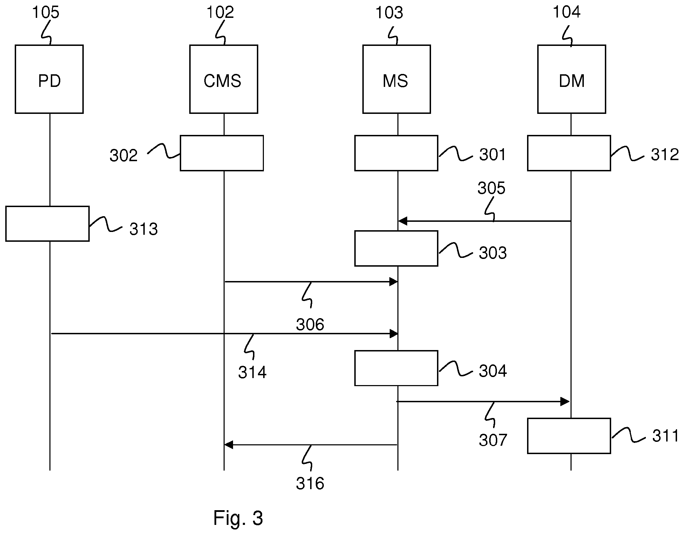

Figur 3 ist ein weiteres Flussdiagramm für einen Nachrichtenfluss zusammen mit Verarbeitungsschritten im Zusammenhang mit dem in Figur 1 dargestellten Datenmanagementsystem und mit einer Dialysebehandlung.

Der Dialysebehandlung geht ein Vorbereitungsschritt 301 zur Vorbereitung einer Dialysebehandlung voraus. In diesem Schritt wird in dem Monitoringsystem 103 ein vorgegebener Datensatz von maschinenbezogenen Behandlungsparametern bereitgestellt, unabhängig von der Behandlung eines bestimmten Patienten. Dabei werden mehrere Datensätze von maschinenabhängigen Parametern zur Verfügung gestellt, jeweils für einen bestimmten Typ einer Dialysemaschine, die in Abhängigkeit von einer Identifizierung des Typs der Dialysemaschine abgelegt und anhand des Typs der Dialysemaschine zugreifbar sind.

Der Dialysebehandlung geht außerdem ein weiterer Vorbereitungsschritt 302 voraus, in dem ein patientenbezogener Datensatzes von patientenbezogenen Behandlungsparametern eines zu behandelnden Patienten, unabhängig von einer für die Behandlung vorgesehenen Dialysemaschine, oder von einem bestimmten Gerätetyp einer Dialysemaschine, in dem Clinical Management System 102 angelegt wird.

Vorzugsweise sind die patientenbezogenen Behandlungsparameter ein Teil einer ärztlichen Verschreibung für den Dialysepatienten.

Der patientenbezogene Datensatz umfasst vorzugsweise eines oder mehrere der folgenden Elemente:
der Art des durchzuführenden Blutbehandlungsverfahrens, einschließlich Hämodialyse, Hämofiltration, Hämodiafiltation, oder Online -Hämodiafiltration,
eine Vorgabe zum Gewichtsmanagement des zu behandelnden Patienten einschließlich eines Trockengewichts oder eines Zielgewichts des zu behandelnden Patienten, sowie eines Schätzwerts für das Kleidungsgewicht,
eine Vorgabe zum Flüssigkeitsmanagement einschließlich einer während der durchzuführenden Behandlung vorgesehenen Flüssigkeitsaufnahme, eine Vorgabe für eines oder mehrere während der Behandlung zu verabreichende Medikamente, eine Vorgabe für eine während der Blutbehandlung vorzunehmendes Antikoagulationsmanagement,
eine Vorgabe für ein während der Dialysebehandlung zu verwendendes Verbrauchsmittel einschließlich eines Typs einer während der Dialysebehandlung zu verwendenden Nadel, einschließlich einer arteriellen Nadel, einer venösen Nadel, eines Typs des während der Dialysebehandlung zu verwendenden Dialysators oder Schlauchsystems einschließlich eins venösen oder arteriellen Blutschlauchsystems, ein während der Dialysebehandlung zu verwendenden Dialysekonzentrat, eine Vorgabe ob die Behandlung als Einnadelbehandlung (Single Needle) oder Mehrnadelbehandlung (Double Needle) durchzuführen ist, eine Vorgabe für den während einer Hämodialysebehandlung einzustellenden Blutfluss, sowie
eine für die Dialysebehandlung vorgesehene Behandlungszeit.

Die patientenbezogenen Behandlungsparameter werden in einem Übertragungsschritt 306 von dem Clinical Management System an das Monitoringsystem übertragen und dort empfangen.

Parallel dazu erfolgt in einem Auswahlschritt 312 die Auswahl der für die Dialysebehandlung vorgesehenen Dialysemaschine 104, etwa indem eine Pflegekraft den Patienten an der vorgesehenen Dialysemaschine anmeldet und dazu vorzugsweise eine Identifizierung oder Authentifizierung des Patienten an der Dialysemaschine durchführt.

Dem Auswahlschritt 312 schließt sich die Übermittlung der Nachricht 305 an, mit der die Dialysemaschine 104 an das Monitoringsystem 103 eine Identifikation sendet, die es erlaubt, den Typ der für die Dialysebehandlung des Patienten vorgesehenen Dialysemaschine 104 zu bestimmen wie z.B. eine Seriennummer der Dialysemaschine 104.

Diese Nachricht enthält außerdem vorzugsweise eine zuvor bestimmte Patientenidentifikation.

Das Monitoringsystem bestimmt anhand der in der Nachricht 305 enthaltenen Information den Typ der für die Behandlung vorgesehenen Dialysemaschine.
Anhand der Identifikation der Dialysemaschine erfolgt die Auswahl eines maschinenbezogenen Datensatzes von maschinenbezogenen Behandlungsparametern für eine bestimmte Dialysebehandlung des Patienten in einem Verarbeitungsschritt 303.
Der maschinenbezogene Datensatz enthält eines oder mehrere der folgenden Elemente:
Vorgaben für während der Blutbehandlung vorzunehmende Messungen des Blutvolumens oder einer Steuerung des Blutvolumens, Vorgaben von Parametern welche in Abhängigkeit von dem Typ der Dialysemaschine unterschiedlich sein können, z.B. eines Ultrafiltrationsprofils, Moduleinstellungen, Vorgaben für die Messung der Rezirkulation oder der Bluttemperatur, einschließlich einer Häufigkeit von Rezirkulationsmessungen oder, Bluttemperaturmessungen, Vorgaben für die Art des Online-Hämodiafltrationsverfahrens (d.h. ob die Zugabe des Substituats blutseitig stromauf des Dialysators erfolgen soll (Pre-dilution), ob die Zugabe stromab des Dialysators erfolgen soll, (Post-dilution), oder ob sie sowohl stromauf als auch stromab des Dialysators erfolgen soll, (Mixed dilution).

In dem Monitoringsystem 103 werden die patientenbezogenen Behandlungsparameter sowie die maschinenbezogenen Behandlungsparameter verwendet, um in einem Verarbeitungsschritt 304 einen Behandlungsdatensatz zu erzeugen, zum Festlegen von Behandlungsparametern für die individuell durchzuführende Behandlung des Patienten.

In dem Monitoringsystem 103 werden außerdem Messdaten von peripheren Geräten 313 wie Waagen oder Blutgasanalysegeräten empfangen (Schritt 314).

Diese Messdaten werden vorzugsweise bei der Erzeugung des Behandlungsdatensatzes berücksichtigt. So kann bei der Festlegung der während einer bestimmten Behandlung zu entziehenden Flüssigkeitsmenge als einer der Behandlungsparameter das Trockengewicht sowie das mit der Waage bestimmte Tagesgewicht eingehen.

Vorzugsweise umfasst der Behandlungsdatensatz einen Steuerungsdatensatz zum Steuern einer Dialysemaschine während der Dialysebehandlung. Das Datenformat des Steuerungsdatensatzes entspricht den Erfordernissen der für die Behandlung vorgesehenen Dialysemaschine.

*Der Behandlungsdatensatz wird vorzugsweise an das Clinical Managementsystem versendet (Schritt 316), um eine Dokumentation der vorgenommenen Behandlung zu dokumentieren.*

Der Behandlungsdatensatz oder Steuerungsdatensatz wird in einem Übertragungsschritt 307 an die ausgewählte Dialysemaschine geschickt und dort empfangen. Die Übertragung des Steuerungsdatensatzes ist vorzugsweise über einen Authentifizierungsmechanismus oder Sicherheitsmechanismus vor Manipulation oder Verfälschung der Daten geschützt. In einer Ausführungsform wird der Steuerungsdatensatz zur Übertragung an die Dialysemaschine 311 in mehrere Übertragungspakete aufgeteilt.

Anschließend wird der Patient an der Dialysemaschine 104 behandelt (Schritt 311), wobei zur Voreinstellung oder zum Steuern der Dialysemaschine 104 während der Dialysebehandlung der Behandlungsdatensatz oder Steuerungsdatensatz verwendet wird.

## Patentansprüche

1. Verfahren zum Bereitstellen von Behandlungsparametern für eine Dialysebehandlung umfassend die folgenden Schritte:
Bereitstellen eines vorgegebenen maschinenbezogenen Datensatzes von maschinenbezogenen Behandlungsparametern, die unabhängig von der Behandlung eines bestimmten Patienten (208, 301) vorgegeben werden,
Breitstellen eines patientenbezogenen Datensatzes von patientenbezogenen Behandlungsparametern eines zu behandelnden Patienten (209, 302), die unabhängig von einer für die Behandlung vorgesehenen Dialysemaschine oder eines Gerätetyps vorgegeben werden, Auswahl eines maschinenbezogenen Datensatzes für eine bestimmte Dialysebehandlung eines Patienten (214, 303), und Verwenden des maschinenbezogenen Datensatzes und des patientenbezogenen Datensatzes zum Erzeugen eines Behandlungsdatensatzes zum Festlegen von Behandlungsparametern für die durchzuführende Dialysebehandlung (204, 304), weiterhin umfassend: Bereitstellen einer Vielzahl von maschinenbezogenen Datensätzen in Abhängigkeit von einem jeweiligen Maschinentyp für eine Vielzahl von Typen von Dialysemaschinen, Bestimmen des Maschinentyps einer für eine bestimmte Behandlung vorgesehene Dialysemaschine und Auswahl des maschinenbezogenen Datensatzes in Abhängigkeit von dem bestimmten Maschinentyp, und wobei der Behandlungsdatensatz einen Steuerungsdatensatz zum Steuern einer Dialysemaschine während der Dialysebehandlung umfasst.

2. Verfahren nach Anspruch 1, wobei der Schritt des Bereitstellens einer Vielzahl von maschinenbezogenen Datensätzen in Abhängigkeit von dem jeweiligen Maschinentyp in einem Monitoringsystem zur Überwachung einer Vielzahl von Dialysemaschinen erfolgt.

3. Verfahren nach einem der vorangehenden Ansprüche, wobei die maschinenbezogenen Behandlungsparameter ausgewählt sind aus einer Gruppe von:
einer Grundform eines Behandlungsprofils einschließlich einer Grundform eines Ultrafiltrationsprofils oder eines Konzentrationsprofils für die Dialysierflüssigkeit, der Patientendatensatz einen Behandlungsparameter einschließlich einer Ultrafiltrationsmenge, oder einer Gesamtkonzentration umfasst, und wobei Behandlungsdatensatz ein während der Behandlung zu verwendendes Behandlungsprofil umfasst, und wobei das zu verwendende Behandlungsprofil aus der Grundform des Behandlungsprofils und dem Behandlungsparameter gebildet wird.

4. Verfahren nach einem der vorangehenden Ansprüche, wobei die maschinenbezogenen Behandlungsparameter ausgewählt sind aus einer Gruppe von: Vorgaben für während der Blutbehandlung vorzunehmende Messungen des Blutvolumens, der Bluttemperatur, oder der Rezirkulation, eine Vorgabe für den Behandlungstyp eines Online- Hämodiafiltrationsverfahrens.

5. Verfahren nach einem der vorangehenden Ansprüche, wobei die patiententenbezogenen Behandlungsparameter ausgewählt sind aus einer Gruppe von:
einer Art des durchzuführenden Behandlungsverfahrens,
einer Vorgabe zum Gewichtsmanagement des zu behandelnden Patienten einschließlich eines Trockengewichts oder eines Zielgewichts des zu behandelnden Patienten,
einer Vorgabe zum Flüssigkeitsmanagement einschließlich einer während der durchzuführenden Behandlung vorgesehenen Flüssigkeitsaufnahme,
einer Vorgabe für eine während der Behandlung vorgesehene Medikamentengabe, einer Vorgabe für eine während der Blutbehandlung vorzunehmende Antikoagulationsbehandlung,
einer Vorgabe für ein während der Dialysebehandlung zu verwendendes Verbrauchsmittel einschließlich eines Typs einer während der Dialysebehandlung zu verwendenden Nadel, eines Typs des während der Dialysebehandlung zu verwendenden Dialysators oder Schlauchsystems oder eines während der Dialysebehandlung zu verwendenden Dialysekonzentrats, eine Vorgabe für den während einer Hämodialysebehandlung einzustellenden Blutfluss,
eine für die Dialysebehandlung vorgesehene Behandlungszeit.

6. Verfahren nach einem der vorangehenden Ansprüche, wobei die patientenbezogenen Behandlungsparameter ein Teil einer ärztlichen Verschreibung für einen Dialysepatienten sind.

7. Verfahren nach einem der vorangehenden Ansprüche, wobei der Schritt des Bereitstellens der patientenbezogenen Behandlungsparameter eine Eingabe in einem Clinical Management System zur Handhabung von patientenbezogener Information umfasst.

8. Verfahren nach Anspruch 3, sowie Anspruch 7, enthaltend die folgenden in dem Monitoringsystem durchgeführten Schritte:
Empfangen der patientenbezogenen Behandlungsparameter von dem Clinical Management System,
Empfangen einer Identifikation eines Typs einer für die Dialysebehandlung vorgesehenen Dialysemaschine, Auswahl des maschinenbezogenen Datensatzes anhand der empfangenen Identifikation des Typs der Dialysemaschine, Absenden des Steuerungsdatensatzes zum Steuern der Dialysemaschine während der Dialysebehandlung an die für die Dialysebehandlung vorgesehene Dialysemaschine.

9. Verfahren nach Anspruch 8, wobei der Steuerungsdatensatz zur Übertragung an die Dialysemaschine in mehrere Übertragungspakete aufgeteilt wird.

10. Verfahren nach Anspruch 8 oder 9, wobei der Steuerungsdatensatz durch einen Authentifizierungsmechanismus vor Manipulation geschützt wird.

11. System aus einem klinischen Informationssystem zur Handhabung patientenbezogener Information und einem Monitoringsystem zur Überwachung einer Vielzahl von Dialysemaschinen und zum Verwalten einer Vielzahl von maschinenbezogenen Datensätzen in Abhängigkeit von dem jeweiligen Maschinentyp,
wobei das klinische Informationssystem Speichermittel zum Breitstellen eines patientenbezogenen Datensatzes von patientenbezogenen Behandlungsdaten eines zu behandelnden Patienten, unabhängig von einer für die Behandlung vorgesehenen Dialysemaschine umfasst und das Monitoringsystem Speichermittel zum Bereitstellen eines Datensatzes von maschinenbezogenen Behandlungsparametern, die unabhängig von der Behandlung eines bestimmten Patienten vorgegeben sind, umfasst sowie eine Berechnungseinheit und Auswahlmittel zur Auswahl eines maschinenbezogenen Datensatzes für eine bestimmte Dialysebehandlung eines Patienten, umfasst sowie Mittel zum Verwenden des maschinenbezogenen Datensatzes und des patientenbezogenen Datensatzes zum Erzeugen eines Behandlungsdatensatzes zum Festlegen von Behandlungsparametern für die durchzuführende Dialysebehandlung, und wobei das Monitoringsystem angepasst ist, reine Vielzahl von maschinenbezogenen Datensätzen in Abhängigkeit von einem jeweiligen Maschinentyp für eine Vielzahl von Typen von Dialysemaschinen bereitzustellen, den Maschinentyp einer für eine bestimmte Behandlung vorgesehenen Dialysemaschine zu bestimmen, und den maschinenbezogenen Datensatz in Abhängigkeit von dem bestimmten Maschinentyp auszuwählen, und wobei der Behandlungsdatensatz einen Steuerungsdatensatz zum Steuern einer Dialysemaschine während der Dialysebehandlung umfasst.

12. System nach Anspruch 11 angepasst zur Durchführung eines Verfahrens nach einem der Ansprüche 2 - 10.

## Claims

1. A method for providing treatment parameters for a dialysis treatment comprising the following steps:
providing a predetermined machine-related dataset of machine-related treatment parameters, which are predetermined independently of the treatment of a specific patient (208, 301),
providing a patient-related dataset of patient-related treatment parameters of a patient (209, 302) to be treated, which treatment parameters are predetermined independently of a dialysis machine or of a device type designated for the treatment, selection of a machine-related dataset for a specific dialysis treatment of a patient (214, 303), and using the machine-related dataset and the patient-related dataset to generate a treatment dataset for determining treatment parameters for the dialysis treatment (204, 304) to be carried out, further comprising: providing a plurality of machine-related datasets as a function of a respective machine type for a plurality of types of dialysis machines, determining the machine type of a dialysis machine designated for a specific treatment, and selection of the machine-related dataset as a function of the specific machine type, and wherein the treatment dataset comprises a control dataset for controlling a dialysis machine during the dialysis treatment.

2. The method according to claim 1, wherein the step of providing a plurality of machine-related datasets as a function of the respective machine type takes place in a monitoring system for monitoring a plurality of dialysis machines.

3. The method according to one of the preceding claims, wherein the machine-related treatment parameters are selected from a group of:
a basic form of a treatment profile including a basic form of an ultrafiltration profile or of a concentration profile for the dialysis fluid, the patient dataset comprises a treatment parameter including an ultrafiltration quantity, or a total concentration, and wherein treatment dataset comprises a treatment profile to be used during the treatment, and wherein the treatment profile to be used is formed from the basic form of the treatment profile and the treatment parameter.

4. The method according to one of the preceding claims, wherein the machine-related treatment parameters are selected from a group of: guidelines for measurements of the blood volume to be performed during the blood treatment, of the blood temperature, or of the recirculation, a guideline for the treatment type of an online hemodiafiltration process.

5. The method according to one of the preceding claims, wherein the patient-related treatment parameters are selected from a group of:
a type of the treatment process to be carried out,
a guideline relating to the weight management of the patient to be treated including a dry weight or a target weight of the patient to be treated,
a guideline relating to the fluid management including a fluid intake designated during the treatment to be carried out,
a guideline for a drug administration designated during the treatment,
a guideline for an anticoagulation treatment to be performed during the blood treatment,
a guideline for a consumable to be used during the dialysis treatment including a type of a needle to be used during the dialysis treatment, of a type of the dialyzer or tube system to be used during the dialysis treatment or of a dialysis concentrate to be used during the dialysis treatment,
a guidelines for the flood flow to be adjusted during a hemodialysis treatment,
a treatment time designated for the dialysis treatment.

6. The method according to one of the preceding claims, wherein the patient-related treatment parameters are a part of a medical prescription for a dialysis patient.

7. The method according to one of the preceding claims, wherein the step of providing the patient-related treatment parameters comprises an input into a clinical management system for handling patient-related information.

8. The method according to claim 3, as well as claim 7, including the following steps carried out in the monitoring system:
receiving the patient-related treatment parameters from the clinical management system,
receiving an identification of a type of a dialysis machine designated for the dialysis treatment, selection of the machine-related dataset by means of the received identification of the type of the dialysis machine, sending the control dataset for controlling the dialysis machine during the dialysis treatment to the dialysis machine designated for the dialysis treatment.

9. The method according to claim 8, wherein the control dataset for transmission to the dialysis machine is divided into a plurality of transmission packets.

10. The method according to claim 8 or 9, wherein the control dataset is protected against manipulation by an authentication mechanism.

11. A system of a clinical information system for handling patient-related information and a monitoring system for monitoring a plurality of dialysis machines and for managing a plurality of machine-related datasets as a function of the respective machine type,
wherein the clinical information system comprises storage means for providing a patient-related dataset of patient-related treatment data of a patient to be treated, independently of a dialysis machine designated for the treatment, and the monitoring system comprises storage means for providing a dataset of machine-related treatment parameters, which are predetermined independently of the treatment of a specific patient, as well as a calculation unit and selection means for selecting a machine-related dataset for a specific dialysis treatment of a patient, as well as means for using the machine-related dataset and the patient-related dataset to generate a treatment dataset for determining treatment parameters for the dialysis treatment to be performed, and wherein the monitoring system is adapted to provide a plurality of machine-related datasets as a function of a respective machine type for a plurality of types of dialysis machines, to determine the machine type of a dialysis machine designated for a specific treatment, and to select the machine-related dataset as a function of the specific machine type, and wherein the treatment dataset comprises a control dataset for controlling a dialysis machine during the dialysis treatment.

12. The system according to claim 11 adapted to carry out a method according to one of claims 2 to 10.

## Revendications

1. Procédé, destiné à mettre à disposition des paramètres de traitement pour un traitement par dialyse, comprenant les étapes suivantes, consistant à :
mettre à disposition un ensemble de données prédéfinies relatif à la machine de paramètres de traitement relatifs à la machine, qui sont prédéfinis indépendamment du traitement d'un certain patient (208, 301),
mettre à disposition un ensemble de données relatives au patient de paramètres de traitement relatifs au patient d'un patient (209, 302) qui doit être traité, qui sont prédéfinies indépendamment d'une machine de dialyse prévue pour le traitement ou d'un type d'appareil, sélectionner un ensemble de données relatives à la machine pour un certain traitement par dialyse d'un patient (214, 303), et utilisation de l'ensemble de données relatives à la machine et de l'ensemble de données relatives au patient pour créer un ensemble de données de traitement, pour fixer des paramètres de traitement pour le traitement par dialyse (204, 304) qui doit être réalisé, comprenant par ailleurs : la mise à disposition d'une pluralité d'ensembles de données relatives à la machine en fonction d'un type de machine respectif pour une pluralité de types de machines de dialyse, et la sélection de l'ensemble de données relatives à la machine en fonction du certain type de machine et l'ensemble de données de traitement comprenant un ensemble de données de commande destinées à commander la machine de dialyse pendant le traitement par dialyse.

2. Procédé selon la revendication 1, l'étape de la mise à disposition d'une pluralité d'ensembles de données relatives à la machine s'effectuant en fonction du type respectif de machine dans un système de surveillance destiné à superviser une pluralité de machines de dialyse.

3. Procédé selon l'une quelconque des revendications précédentes, les paramètres de traitement relatifs à la machine étant sélectionnés dans le groupe comprenant :
une forme basique d'un profil de traitement incluant une forme basique d'un profil d'ultrafiltration ou d'un profil de concentration pour le liquide de dialyse, le jeu de données du patient comprenant un paramètre de traitement incluant une quantité d'ultrafiltration, ou une concentration totale, et l'ensemble de données de traitement comprenant un profil de traitement qui doit être utilisé pendant le traitement, et le profil de traitement qui doit être utilisé étant créé à partir de la forme basique du profil de traitement et du paramètre de traitement.

4. Procédé selon l'une quelconque des revendications précédentes, les paramètres de traitement relatifs à la machine étant sélectionnés dans un groupe comprenant : des prescriptions de mesure du volume sanguin, de la température sanguine ou de la recirculation qu'il convient d'effectuer pendant le traitement sanguin, une prescription pour le type de traitement d'un procédé d'hémodiafiltration en ligne.

5. Procédé selon l'une quelconque des revendications précédentes, les paramètres de traitement relatifs au patient étant sélectionnés dans le groupe comprenant :
un type du procédé de traitement qui doit être réalisé,
une prescription pour la gestion du poids du patient qui doit être traité, incluant un poids sec ou un poids cible du patient qui doit être traité,
une prescription de gestion de liquide, incluant une prise de liquide prévue pensant le traitement qui doit être réalisé,
une prescription pour l'administration d'un médicament prévue pendant le traitement,
une prescription pour un traitement anticoagulant qui doit être effectué pendant le traitement du sang,
une prescription d'un produit de consommation qui doit être utilisé pendant le traitement par dialyse incluant un type d'une aiguille qui doit être utilisée pendant le traitement par dialyse, un type du dialyseur qui doit être utilisé pendant le traitement par dialyse ou d'un concentré pour dialyse qui doit être utilisé pendant le traitement par dialyse,
une prescription pour le flux sanguin qui doit être réglé pendant le traitement par hémodialyse,
un temps de traitement prévu pour le traitement par dialyse.

6. Procédé selon l'une quelconque des revendications précédentes, les paramètres de traitement relatifs au patient étant une partie d'une ordonnance médicale pour un patient à dialyser.

7. Procédé selon l'une quelconque des revendications précédentes, l'étape de la mise à disposition des paramètres de traitement relatifs au patient comprenant une saisie dans un Clinical Management System pour la manipulation d'informations relatives au patient.

8. Procédé selon la revendication 3, ainsi que selon la revendication 7, contenant les étapes suivantes, réalisées dans le système de surveillance, consistant à :
réceptionner les paramètres de traitement relatifs au patient de la part du Clinical Management System,
réceptionner une identification d'un type d'une machine de dialyse prévue pour le traitement par dialyse, sélectionner l'ensemble de données relatives à la machine à l'aide de l'identification réceptionnée du type de la machine de dialyse, envoyer l'ensemble de données de commande, destiné à commander la machine de dialyse pendant le traitement par dialyse à la machine de dialyse prévue pour le traitement par dialyse.

9. Procédé selon la revendication 8, pour la transmission à la machine de dialyse, l'ensemble de données de commande étant réparti en plusieurs paquets de transmission.

10. Procédé selon la revendication 8 ou 9, l'ensemble de données de commande étant protégé contre une manipulation par un mécanisme d'authentification.

11. Système, composé d'un système d'information clinique, destiné à manipuler une information relative à un patient et d'un système de surveillance destiné à superviser une pluralité de machines de dialyse et à gérer une pluralité d'ensembles de données relatives à la machine, en fonction du type respectif de machine,
le système d'information clinique comprenant des moyens de mémoire, destinés à mettre à disposition un ensemble de données relatives au patient de données de traitement relatives au patient d'un patient qui doit être traité, indépendamment d'une machine de dialyse prévue pour le traitement, et le système de surveillance comprenant des moyens de mémoire, destinés à mettre à disposition un ensemble de données de paramètres de traitement relatifs à la machine, qui sont prescrits indépendamment d'un traitement d'un certain patient et comprenant une unité de calcul et des moyens de sélection, destinés à sélectionner un ensemble de données relatives à la machine pour un certain traitement par dialyse d'un patient et comprenant des moyens destinés à utiliser l'ensemble de données relatives à la machine et l'ensemble de données relatives au patient, pour créer un ensemble de données de traitement, pour fixer des paramètres de traitement pour le traitement par dialyse qui doit être réalisé, et le système de surveillance étant adapté pour mettre à disposition une pluralité d'ensembles de données relatives à la machine, en fonction d'un type respectif de machine pour une pluralité de types de machines de dialyse, pour déterminer le type de machine d'une machine de dialyse prévue pour un certain traitement et pour sélectionner l'ensemble de données relatives à la machine en fonction du type de machine déterminé et comprenant l'ensemble de données de commande destinées à commander une machine de dialyse pendant le traitement par dialyse.

12. Système selon la revendication 11, adapté pour réaliser un procédé selon l'une quelconque des revendications 2 à 10.
